(19) Europäisches Patentamt / European Patent Office / Office européen des brevets

(11) **EP 4 426 368 B1**

(12) **EUROPEAN PATENT SPECIFICATION**

(45) Date of publication and mention
of the grant of the patent:
**26.11.2025 Bulletin 2025/48**

(21) Application number: **22802085.5**

(22) Date of filing: **11.10.2022**

(51) International Patent Classification (IPC):
*A61M 1/16* (2006.01)      *A61M 1/36* (2006.01)
*A61M 1/34* (2006.01)      *A61P 13/12* (2006.01)

(52) Cooperative Patent Classification (CPC):
**A61M 1/3609; A61M 1/3437; A61M 1/3458;**
**A61P 13/12;** A61M 1/3434; A61M 1/3639;
A61M 2205/3334; A61M 2230/06; A61M 2230/207;
A61M 2230/30

(86) International application number:
**PCT/EP2022/078290**

(87) International publication number:
**WO 2023/078648 (11.05.2023 Gazette 2023/19)**

(54) **APPARATUS FOR EXTRACORPOREAL BLOOD TREATMENT**

VORRICHTUNG ZUR EXTRAKORPORALEN BLUTBEHANDLUNG

APPAREIL DE TRAITEMENT EXTRACORPOREL DU SANG

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.11.2021 IT 202100028082**

(43) Date of publication of application:
**11.09.2024 Bulletin 2024/37**

(73) Proprietor: **Gambro Lundia AB
226 43 Lund (SE)**

(72) Inventor: **FORSAL, Innas
21445 Malmö (SE)**

(74) Representative: **PGA S.p.A., Milano, Succursale
di Lugano
Via Castagnola, 21c
6900 Lugano (CH)**

(56) References cited:
**CA-A1- 2 824 391      US-A1- 2021 106 738**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an apparatus for extracorporeal blood treatment. The present disclosure also relates to a method (which is excluded from the scope of the present invention; this applies to any occurrence of the term "method for") for controlling a patient fluid removal in an apparatus for extracorporeal blood treatment.

Extracorporeal blood treatment involves removing blood from a patient, treating the blood externally to the patient, and returning the treated blood to the patient. Extracorporeal blood treatment is typically used to extract undesirable matter or molecules from the patient's blood and add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance, to remove excess body fluids or to perform extracorporeal gas exchange processes, for example.

The present invention refers to the treatment of fluid overload of patients through extracorporeal blood treatments, in particular but not exclusively in continuous renal replacement therapy (CRRT) systems. CRRT systems are configured for delivering treatments designed for patients versing in acute states of illness and who have temporarily lost their kidney function in its entirety. CRRT monitors should be able to deliver various therapies, e.g.: ultrafiltration (UF), slow continuous ultrafiltration (SCUF), continuos veno venous hemofiltration (CCVH), continuos veno venous hemodiafiltration (CVVHDF).

The invention may also be applied to chronic (long-term) hemodialysis (HD) therapy systems. Chronic (long-term) hemodialysis (HD) therapy systems are used to treat patients with chronic kidney failure. HD therapy is usually performed several times a week (e.g. 3 times a week) and each treatment has an average duration of some hours (e.g. 3.5 - 4.5 hours).

Notably, the present invention may also be used in connection with Congestive Heart Failure (CHF) treatments; congestive heart failure (CHF) is a set of manifestations caused by the failure of the heart's function as a pump supporting the blood flow through the body. Filling dysfunction and high intracardiac pressure may result in the buildup of fluid in the veins and tissues. This manifests as water retention and swelling due to the buildup of liquid (edema), collectively referred to as congestion. Ultrafiltration treatments are used to remove excess water, particularly in case diuretics have no or little effects or when the patient has no or residual renal function.

BACKGROUND OF THE INVENTION

Conventional treatment of fluid overload is the usage of diuretic and measuring the patient's weight until a goal weight is achieved.

When a diuretic is administered, fluid is recruited directly from the vascular volume via the kidney and if this rate exceeds the rate that the resultant oncotic pressure can recruit fluid from the interstitial space (splanchnic and somatic volumes), the volume in the vascular space is depleted. This replenishment rate is known as the PRR (Plasma Refill Rate).

The problem with diuretics is that, if the patient has no urine production, no fluid will be removed. Furthermore, if the fluid is located peripherally in the interstitial space, the increase of diuretics does not improve excess fluid removal but may cause hypotension to the patient and/or renal failure.

Continuous renal replacement therapy (CRRT) may also be used for treatment of fluid overload. These are quite long treatments with slow but prolonged removal of liquids.

Unfortunately, also CRRT presents the same problem related to the peripherally located fluid. There have been tricks that Medical Doctors have done to get better removal of excess fluids through CRRT and two of these is usage of albumin as an osmotic agent to force fluid into the central circulation and diuretics infusion or oral administration of diuretics to remove fluid through urine.

Document US 2007/0055198 is also known, which discloses a blood volume control method comprising: monitoring a condition of a patient indicative of blood volume in the patient, determining that the condition indicates a low blood volume in the patient; infusing an infusion solution into the patient in response to the determination of a low blood volume and adjusting the infusion to maintain the monitored condition at a predetermined value. The method disclosed in US 2007/0055198 also provides for infusing furosemide in the patients' peripheral blood vessels in the forearm or centrally in the venous system and for increasing artificially the Plasma Refill Rate (PRR) by the infusion of a Hypertonic Saline Solution or other such blood volume expander such as a dextrose solution, sodium bicarbonate solution or albumin using a standard syringe and IV needle.

Document US20211106738 deals with a medical apparatus far extracorporeal treatment of blood comprising a treatment unit, a blood circuit coupled to the treatment unit and comprising a blood removal line and a blood return line connectable to a vascular system of a patient. The blood pump is coupled ta a pump section of the blood circuit to deliver a blood flow rate. An effluent line is connected to a treatment unit and an effluent fluid flow is delivered by an effluent pump. The control unit receives input apparatus parameters and input patient parameters during the treatment.

CA2824391 is a prior art dealing with compositions comprising crosslinked cation-binding polymer to treat renal disease.

There is a desire to further improve known extracorporeal blood treatments for the removal of excess fluid using diuretics and/or osmotic agents.

An aim of the present invention is therefore to provide for an apparatus for extracorporeal blood treatment and a method for controlling a patient fluid removal that allow to achieve a better fluid removal and a better treatment to the patient.

It is an aim of the present invention to provide for an apparatus for extracorporeal blood treatment and a method for controlling a patient fluid removal that allow to reduce risks of criticalities in patients undergoing treatment.

It is an aim of the present invention to provide a method for controlling a patient fluid removal which is automatically executed during an extracorporeal blood treatment.

It is an aim of the present invention to tailor the treatment and in particular the treatment of fluid overload to each specific patient and to each specific extracorporeal blood treatment.

It is a further aim of the present invention to improve the comfort of patients undergoing treatment.

It is a further aim of the present invention to reduce the workload of the clinical staff and/or of the Medical Doctors due in particular to problems linked to fluid removal.

## SUMMARY

An apparatus according to one or more of the appended claims attains at least one of the above-indicated aims.

The applicant noted that known extracorporeal blood treatments for the removal of excess fluid using diuretics and/or osmotic agents may be improved by tailoring the infusion of said diuretics and/or osmotic agents to the specific patient and type of extracorporeal blood treatment in order to achieve an improved fluid removal.

The present invention provides an extracorporeal blood treatment apparatus and a method for controlling a patient fluid removal which are able to automatically command the apparatus and control an infusion of a diuretic and/or of an osmotic agent as a function of one or more input patient parameters and/or one or more apparatus parameters.

An apparatus according to the invention is recited in apparatus claim 1.

Further characteristics of the present invention will better emerge from the detailed description that follows of some embodiments of the invention, illustrated by way of non-limiting examples in the accompanying figures.

## DESCRIPTION OF THE DRAWINGS

The description will now follow, with reference to the appended figures, provided by way of non-limiting example, in which:

- Figure 1 schematically shows an extracorporeal blood treatment apparatus according to the invention;
- Figures 2 is a different embodiment of the extracorporeal blood treatment apparatus of the invention;
- Figure 3 is a flow chart of an example of a procedure implemented by the apparatus of the invention;
- Figure 4 is a flow chart of another example of a procedure implemented by the apparatus of the invention;
- Figure 5 is a flow chart of another example of a procedure implemented by the apparatus of the invention.

## DETAILED DESCRIPTION

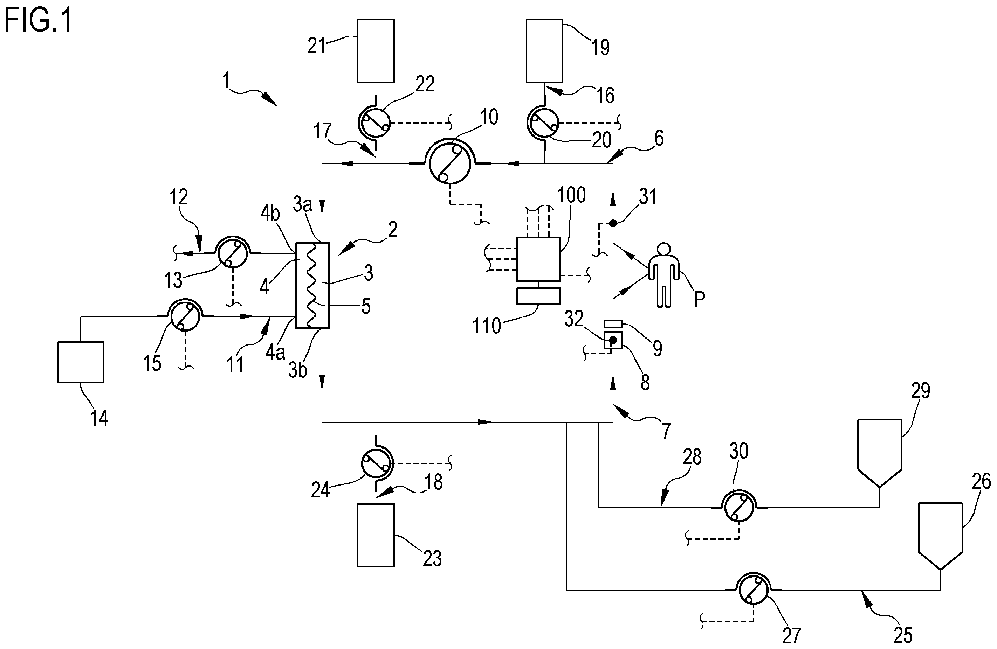

An apparatus 1 for extracorporeal blood treatment is schematically represented in Figure 1. The apparatus 1 may be a continuous renal replacement therapy (CRRT) apparatus for intensive care treatments, for instance configured to deliver various therapies, like CCVH, CVVHDF, SCUF.

The apparatus 1 comprises a treatment or filtration unit 2 having a primary chamber 3 and a secondary chamber 4 separated by a semipermeable membrane 5. Depending upon the treatment, the semipermeable membrane 5 of the treatment unit 2 may be selected to have different properties and performances.

A blood circuit is coupled to the primary chamber 3 of the treatment unit 2. The blood circuit comprises a blood removal line 6 connected to an inlet 3a of the primary chamber 3, a blood return line 7 connected to an outlet 3b of the primary chamber 3. The blood removal line 6 and blood return line 7 are configured for connection to a cardiovascular system of a patient "P".

In use, the blood removal line 6 and the blood return line 7 are connected to a needle or to a catheter or other access device which is then placed in fluid communication with the patient "P" vascular system, such that blood may be withdrawn through the blood removal line 6, flown through the primary chamber 3 and then returned to the patient's vascular system through the blood return line 7.An air separator, such as a deaeration chamber 8, may be present on the blood return line 7. Moreover, a monitor valve 9 may be present on the blood return line 7, downstream the deaeration chamber 8.

The blood flow through the blood circuit is controlled by a blood pump 10, for instance a peristaltic blood pump, acting either on the blood removal line 6 or on the blood return line 7. The embodiment of Figure 1 shows the blood pump 10 coupled to a pump section of the blood removal line 6.

A dialysis circuit is connected to the secondary chamber 4 of the filtration unit 2 and comprises a dialysis line 11 connected to an inlet 4a of the secondary chamber 4 and an effluent line 12 connected to an outlet 4b of the secondary chamber 4 and to a drain, not shown.

An effluent pump 13 is located on the effluent line 12 and is able to recall fluid from the second chamber 4. The dialysis line 11 is connected to a source 14, e.g. a bag or a preparation device, of fresh dialysis fluid and a dialysis pump 15 is located on the dialysis line 11 and is able to pump fluid to the second chamber 4.

The apparatus 1 further comprises an infusion circuit comprising at least one infusion line. The infusion circuit shown in the embodiment of figure 1 comprises a pre-blood pump line 16, a pre-infusion line 17 and a post-infusion line 18.

The pre-blood pump line 16 is connected to the blood removal line 6 upstream of the blood pump 10 and to a first source 19 of infusion fluid, e.g. a bag. A pre-blood pump 20 is located on the pre-blood pump line 16 and is able to pump fluid from the first source 19 to the blood circuit.

The pre-infusion line 17 is connected to the blood removal line 6 downstream of the blood pump 10 and upstream of the treatment unit 2 and to a second source 21 of infusion fluid, e.g. a bag. A pre-infusion pump 22 is located on the pre-infusion line 17 and is able to pump fluid from the second source 21 to the blood circuit.

The post-infusion line 18 is connected to the blood return line 7 downstream of the treatment unit 2 and to a third source 23 of infusion fluid, e.g. a bag. A post-infusion pump 24 is located on the post-infusion line 18 and is able to pump fluid from the third source 23 to the blood circuit.

The apparatus 1 may also comprise one or more auxiliary line/s, not shown, connected to the blood circuit and to a source of at least one compensation substance, such as potassium or bicarbonate, and a pump or syringe configured to deliver a flow rate of the compensation substance, such as potassium or bicarbonate.

A diuretic line 25 is connected to a source 26 of at least one diuretic and to the blood circuit. A diuretic pump 27 is configured to be coupled to a pump section of the diuretic line 25 to deliver a diuretic flow rate "$Q_d$".

The diuretic may be or may comprise furosemide, torsemide or bumetanide, ethacrynic acid, metolazone, chlorthalidone, hydrochlorothiazide, chlorothiazide, spironolactone, eplererone. For instance, the diuretic may be a solution of sodium chloride mixed with furosemide.

An osmotic agent line 28 is connected to a source 29 of at least one osmotic agent and to the blood circuit. An osmotic agent pump 30 is configured to be coupled to a pump section of the osmotic agent line 28 to deliver an osmotic agent flow rate "$Q_{oa}$".

The osmotic agent may be or may comprise albumin, amino acid, mannitol, hypertonic saline, glucose solution.

A control unit 100 is connected and controls the blood pump 10, the effluent pump 13, the dialysis pump 15, the pre-blood pump 20, the pre-infusion pump 22 and the post-infusion pump 24 to regulate a blood flow rate "$Q_b$" in the blood circuit, a dialysis flow rate "$Q_{dial}$" crossing the dialysis line 11, an effluent flow rate "$Q_{eff}$" crossing the effluent line 12, an infusion flow rate "$Q_{pbp}$" crossing the pre-blood pump line 16, an infusion flow rate "$Q_{pre}$" crossing the pre-infusion line 17, an infusion flow rate "$Q_{post}$" crossing the post-infusion line 18.

Through the control of the dialysis flow rate "$Q_{dial}$" crossing the dialysis line 11 and/or of the effluent flow rate "$Q_{eff}$" crossing the effluent line 12, the control unit 100 is also configured to control/regulate a filtration flow rate "$Q_{fil}$" in the treatment unit 2 and/or a patient fluid removal rate "$Q_{pfr}$".

The control unit 100 also controls the diuretic pump 27 and the osmotic agent pump 30 to control respectively the diuretic flow rate "$Q_d$" and the osmotic agent flow rate "$Q_{oa}$".

Flow rate sensors, not shown in the attached drawings, may be placed on the blood circuit, on the dialysis line 11, on the effluent line 12, on the infusion lines 16, 17, 18, on the diuretic line 25, on the osmotic agent line 28 and connected to the control unit 100 to detect and/or control the flow rates of blood and fluids in the apparatus 1 (i.e. blood flow rate "$Q_b$", dialysis flow rate "$Q_{dial}$", effluent flow rate "$Q_{eff}$", infusion flow rates "$Q_{pbp}$", "$Q_{pre}$", "$Q_{post}$", diuretic flow rate "$Q_d$", osmotic agent flow rate "$Q_{oa}$" and also filtration flow rate "$Q_{fil}$" and patient fluid removal rate "$Q_{pfr}$") .

An access pressure sensor 31 is placed on the blood removal line 6 and is connected to the control unit 100 to monitor an access pressure "$P_{acc}$". A return pressure sensor 32 is placed on the blood return line 7, for instance coupled to the deaeration chamber 9, and is connected to the control unit 100 to monitor a return pressure "$P_{ret}$".

Sensors are also provided to detect measured values of patient parameters, such as heart rate "HR", blood pressure "BP", urine output "UO", consciousness level "CL", body weight "BW", creatinine "Cr", hematocrit "Htc", level of electrolytes. These sensors may be known and commonly used.

For instance, a heart rate sensor may be: a patch, a wristband or an armband, a chest strap, a wearable electrode based ECG. A weight scale may be used to measure the body weight "BW". An urometer may be used for measuring urine output "UO". An optical sensor may be used to measure hematocrit "Htc". A digital blood pressure on the wrist, on the finger or upper arm of the patient "P", may be used to monitor patient blood pressure "BP". Saturation devices may also be used to retrieve information about heart rate that can indicate low blood pressure, since heart rate increases when blood pressure is getting lower (this may allow to sense a blood pressure decrease before it actually occurs). Temperature sensor to detect temperature at extremities of the patient may be used to infer blood pressure, since a low temperature means low blood pressure. For consciousness, a BIS monitor (bispectral index) may be used.

These sensors for detecting patient parameters may be connected to the control unit 100 to transmit values to the control unit 100 during the extracorporeal blood treatment (e.g. urine output "UO" and/or consciousness level "CL" and/or heart rate "HR" and/or hematocrit "Htc"). In this case, these patient parameters are measured continuously and continuously received by the control unit 100.

The sensors for detecting patient parameters may also be part of other devices which show the values such that the medical staff may enter them in the control unit 100. One or more of the patient parameters may be laboratory values (e.g. creatinine "Cr" or body weight "BW" or blood pressure "BP"). In these cases, these patient parameters are entered into the control unit 100 one or more times during the treatment or before the treatment starts.

The control unit 100 may be an electronic control unit comprising at least a CPU, a memory and input/output devices. The control unit 100 comprises or is connected to an interface 110 configured to display data and/or allow a user to input data. For instance, the interface comprises a display, e.g. a touch screen, and/or buttons or a keyboard.

The apparatus 1 may comprise a treatment machine 200 and an integrated disposable set configured to be coupled to the treatment machine.

The treatment machine comprises the cited blood pump 10, effluent pump 13, dialysis pump 15, pre-blood pump 20, pre-infusion pump 22, diuretic pump 27, osmotic agent pump 30, control unit 100 with the interface 110, flow rate sensors. The treatment machine may comprise also the access pressure sensor 31 and the return pressure sensor 32 or the access pressure sensor 31 and the return pressure sensor 32 may be part of the integrated disposable set. The treatment machine comprises also all the other elements and/or devices configured to receive and hold parts of the integrated disposable set.

The integrated disposable set comprises the treatment unit 2, the blood circuit, the effluent line 12, the dialysis line 11, the infusion lines 16, 17, 18, the diuretic line 25 and the osmotic agent line 28 which are grouped together.

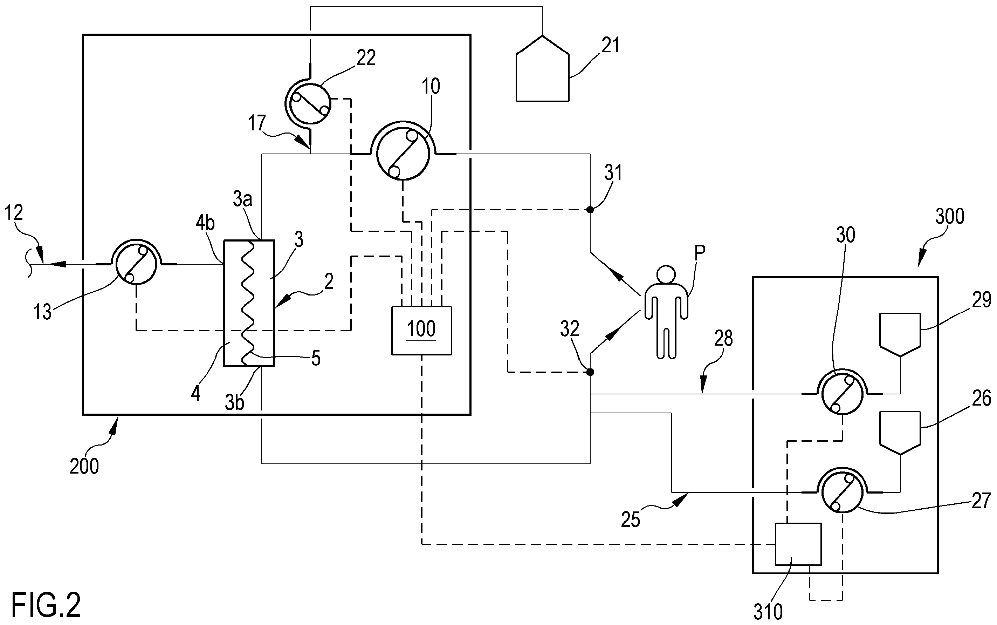

In another embodiment, shown schematically in figure 2, the diuretic pump 27 and the osmotic agent pump 30 are part of an independent device 300 and not of the treatment machine 200. The treatment machine 200 comprises the blood pump 10, the effluent pump 13, the pre-infusion pump 22 and the control unit 100 as detailed above regarding figure 1. The independent device 300 comprises the diuretic pump 27 and the osmotic agent pump 30. The diuretic pump 27 and the osmotic agent pump 30 may be connected and controlled by a control unit 310 of the independent device 300 and this control unit 310 of the independent device 300 is connected to the control unit 100 of the treatment machine 100.

In other embodiments, not shown, the diuretic line 25 and the osmotic agent line 28 may be connected directly to the vascular system of the patient "P" instead of to the blood circuit.

In a further embodiment, the treatment machine 200 may include the blood circuit (blood removal line 6 and blood return line 7), the blood pump 10, the effluent line 12, the effluent pump 13, the treatment unit 2, and the control unit 100 as detailed above regarding figure 2. The here described treatment machine 200 is configured for ultrafiltration (or SCUF) in the field of e.g., congestive heart failure treatments. In this specific CHF treatment, the blood removal line 6 and blood return line 7 may be connected to peripheral veins of the patient to (e.g., slowly) remove excess water. Required infusions (e.g., diuretics and/or osmotic agents) may be connected directly to the vascular system of the patient "P" instead of to the blood circuit or to the blood circuit if required/proper).

The control unit 100 is configured and/or programmed for receiving one or more input patient parameter and/or one or more input apparatus parameter and for driving, during the extracorporeal blood treatment, the diuretic pump 27 and the osmotic agent pump 30 as a function of the input patient parameter/s and/or input apparatus parameter/s, to make sure that the more optimal treatment is given to the patient "P" to achieve the more optimal fluid removal.

The control unit 100 is therefore configured for implementing a script or algorithm and a method for controlling a patient fluid removal in the apparatus for extracorporeal blood treatment.

The input patient parameters and the input apparatus parameters may be prescription values and/or values measured through the sensors and/or devices previously listed.

According to some embodiments, the control unit 100 may be configured and/or programmed for monitoring the measured values over time and driving the diuretic pump and/or the osmotic agent pump as a function of variation over time of the measured values.

According to some embodiments, the control unit is configured and/or programmed for driving the diuretic pump and/or the osmotic agent pump as a function of the prescription value of the input patient parameter/s and/or the input apparatus parameter/s, for instance as a function of a comparison between said prescription value with the measured values of the input patient parameter and/or the input apparatus parameter.

According to examples of the procedure performed by the control unit 100 or of the method, the following input parameters.

Prescription values

| WL | Total patient weight loss or fluid removal goal |
|---|---|
| T | Total treatment time |

Types of diuretic and osmotic agents

Diuretic: e.g. furosemide
Osmotic agent: e.g. albumin

Measured values

Input apparatus parameters

| $Q_d$ | diuretic flow rate |
|---|---|
| $Q_{oa}$ | osmotic agent flow rate |
| $Q_b$ | blood flow rate |
| $Q_{pbp}$ | infusion flow rate crossing pre-blood pump line |
| $Q_{pre}$ | infusion flow rate crossing pre-infusion line |
| $Q_{post}$ | infusion flow rate crossing post-infusion line |
| $Q_{dial}$ | dialysis flow rate |
| $Q_{eff}$ | effluent flow rate |
| $Q_{fil}$ | filtration flow rate |
| $Q_{pfr}$ | patient fluid removal rate |
| $P_{acc}$ | access pressure |
| $P_{ret}$ | return pressure |

wherein $Q_{eff}$ - $Q_{dial}$ + $Q_{fil}$ = $Q_{dial}$ + $Q_{pfr}$ + $Q_{pbp}$ + $Q_{pre}$ + $Q_{post}$ if $Q_o$, and $Q_d$ are negligible.

Input patient parameters

| UO | urine output (measured continuously) |
|---|---|
| CL | consciousness level (measured continuously) |
| HR | heart rate (measured continuously) |
| Htc | hematocrit |
| BW | Body weight (lab value) |
| BP | Blood pressure (lab value) |
| Cr | Creatinine (lab value) |

At the start of the treatment, the following parameters are set as a function of the total patient weight loss or fluid removal goal "WT" and the total treatment time "T".

| $Q_{bset}$ | set blood flow rate |
|---|---|
| $Q_{dialset}$ | set dialysis flow rate |
| $Q_{effset}$ | set effluent flow rate |
| $Q_{dset}$ | set diuretic flow rate |
| $P_{acc\ high}$ | set high access pressure threshold |
| $P_{acc\ low}$ | set low access pressure threshold |
| $P_{ret\ low}$ | set low return pressure threshold |
| $BP_{thr}$ | set blood pressure threshold |
| $Htc_{thr}$ | set hematocrit threshold |

(continued)

| | |
|---|---|
| Cr$_{thr}$ | set creatinine threshold |

At the start of the treatment, the following parameters may be set equal to zero.

$$Q_{oa} = 0$$

$$Q_{pbp} = 0$$

$$Q_{pre} = 0$$

$$Q_{post} = 0$$

Therefore: $Q_{fil} = Q_{eff} - Q_{dial}$

The script or algorithm in the control unit 100 may be able to reach the fluid removal goal "WT" at set total treatment time "T" if no problems occur in regards of the access pressure "P$_{acc}$" and the blood pressure "P$_{acc}$". If problems occur with the access pressure "P$_{acc}$" and the blood pressure "P$_{acc}$", the filtration flow rate "Q$_{fil}$" that may be set is limited and the time to reach the goal will be longer. The script may also consider stops such as pauses in treatment, bag changes, other alarms but also recirculation etc.

The following are examples of the method implemented by the control unit 100.

If the access pressure "P$_{acc}$" is above the high access pressure threshold "P$_{acc\,high}$", for instance of 250 mmHg (1 mmHg=133 Pa) (e.g. high access pressure indicates high blood volume), or within an access pressure range, for instance between 100 mmHg and 160 mmHg, commanding the effluent pump 13 to increase the effluent flow rate "Q$_{eff}$" and the filtration flow rate "Q$_{fil}$".

If the access pressure "P$_{acc}$" is below the low access pressure threshold "P$_{acc\,low}$" (e.g. access pressure that is too low due to hypotension or either there is some hindrance in the blood removal line 6 (due for instance to folding of the catheter or kinked or twisted) or it is too close to the vascular wall or any other problem where the flow is not working properly due to the access not working), commanding the blood pump 10 to reduce the blood flow rate "Q$_b$" and/or commanding the effluent pump 13 to reduce the effluent flow rate "Q$_{eff}$" and the filtration flow rate "Q$_{fil}$" and/or commanding the osmotic agent pump 30 and/or the diuretic pump 27 to reduce or increase the diuretic flow rate "Q$_d$" and/or the osmotic agent flow rate "Q$_{oa}$".

The diuretic flow rate may be between 40 mg/h and 120 mg/h (4 mg/minute) and the osmotic agent flow rate may be between 10 mg/h and 150 mg/h.

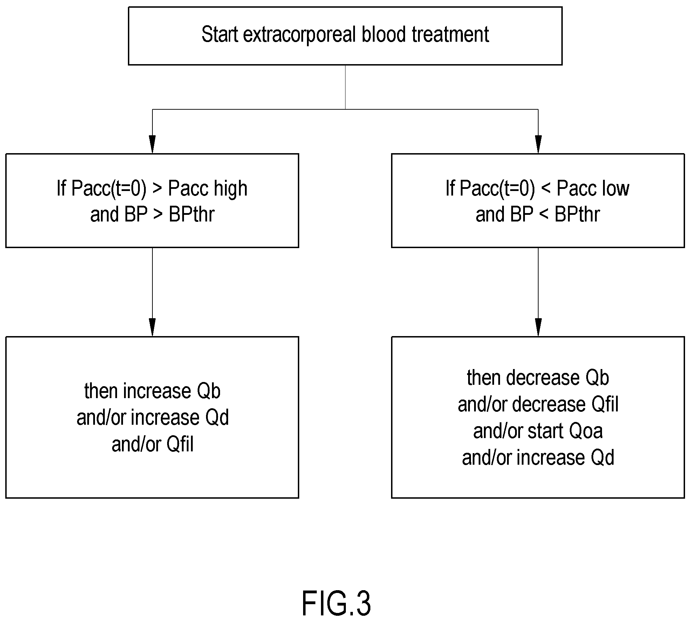

Referring to the flow chart of figure 3, if P$_{acc}$(t=0, i.e. at the start of the treatment) > P$_{acc\,high}$ and BP is in an acceptable range (e.g. > BP$_{thr}$ = 100 mmHg systolic), then increase Q$_b$ and/or increase Q$_d$ and/or Q$_{fil}$.

If P$_{acc}$(t=0) < P$_{acc\,low}$ and BP < BP$_{thr}$ = 100 mmHg systolic, then decrease Q$_b$ and/or decrease Q$_{fil}$ and/or start Q$_{oa}$ (albumin) and/or increase Q$_d$ to increase blood pressure (figure 3).

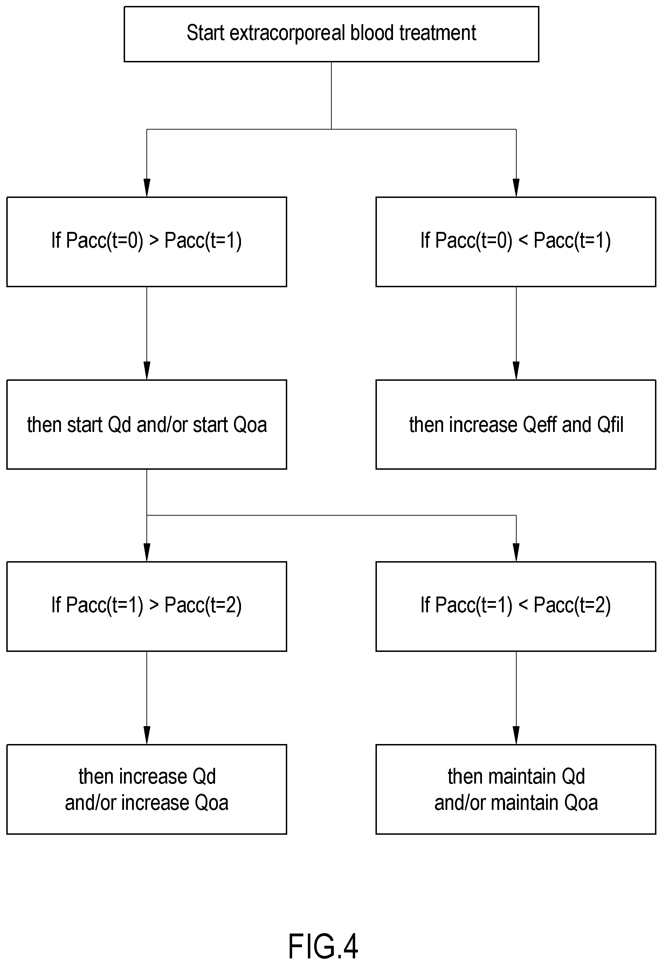

Referring to the flow chart of figure 4, if the access pressure "P$_{acc}$" decreases over time (e.g. if the access pressure at instant t=0 is greater than the access pressure at instant t=1), meaning that blood is more concentrated, i.e. there is less fluid in blood, then starting the diuretic pump 27 and/or the osmotic agent pump 30 to deliver the diuretic flow rate "Q$_d$" and/or the osmotic agent flow rate "Q$_{oa}$"; the osmotic agent forces fluid into the central circulation and diuretic infusion removes fluids through urine. One of the positive aspects of diuretics, such as furosemide, is that they can help remove some fluid in the lungs. If the access pressure "P$_{acc}$" keeps on decreasing over time (e.g. the access pressure at instant t=1 is greater than the access pressure at instant t=2), meaning that no further fluid in blood is available or not high enough concentration of diuretics is present to increase the plasma, then starting the osmotic agent pump 30 and/or the diuretic pump 27 or commanding the diuretic pump 27 to increase the diuretic flow rate "Q$_d$" and/or commanding the osmotic agent pump 30 to increase the osmotic agent flow rate "Q$_{oa}$". If the access pressure "P$_{acc}$" starts increasing over time (e.g. if the access pressure at instant t=2 is greater than the access pressure at instant t=1), meaning that a good result has been achieved, then commanding the diuretic pump 27 to maintain the diuretic flow rate "Q$_d$" and/or commanding the osmotic agent pump 30 to maintain the osmotic agent flow rate "Q$_{oa}$" (figure 3).

If the access pressure "P$_{acc}$" increases over time (e.g. if the access pressure at instant t=1 is greater than the access pressure at instant t=0), taking no action or commanding the effluent pump 13 to increase the effluent flow rate "Q$_{eff}$" and the filtration flow rate "Q$_{fil}$", in order to increase fluid removal from patient "P". Since good conditions are present, the filtration flow rate "Q$_{fil}$" may be increased to remove more fluid faster (figure 4).

If the blood pressure "BP" is within a blood pressure range, then increase the blood flow rate "Q$_b$", increase the diuretic flow rate "$Q_d$" and increase the effluent flow rate "$Q_{eff}$" and the filtration flow rate "$Q_{fil}$". Since good conditions are present, the filtration flow rate may be increased to remove more fluid faster.

If the blood pressure "BP" is below a blood pressure threshold, start the osmotic agent pump 30 or commanding the osmotic agent pump 30 to increase the osmotic agent flow rate "$Q_{oa}$" till the blood pressure "BP" is stabilized and then starting the diuretic pump 27 or commanding the diuretic pump 27 to increase the diuretic flow rate "$Q_d$".

If the blood pressure "BP" is low, i.e. below a blood pressure threshold, and the access pressure "$P_{acc}$" is outside an access pressure range, the infusion flow rate is started, e.g. of saline or glucose. Some patients may have the contradicting problem that the intravascular water concentration is low but there is an increased amount of solution in tissue (extracellular). In these conditions, addition of solution and at the same time removal of solution may be needed.

If the creatinine "Cr" is outside a creatinine range (increase in creatinine may indicate risk for damage on the kidneys) and/or the hematocrit "Htc" is outside a hematocrit range (risk for clotting), then a less aggressive treatment is performed. For instance, the blood flow rate "$Q_b$" is reduced and/or the effluent flow rate "$Q_{eff}$" and the filtration flow rate "$Q_{fil}$" are reduced while the diuretic flow rate "$Q_d$" and/or the osmotic agent flow rate "$Q_{oa}$" are reduced or increased, depending on the specific circumstances.

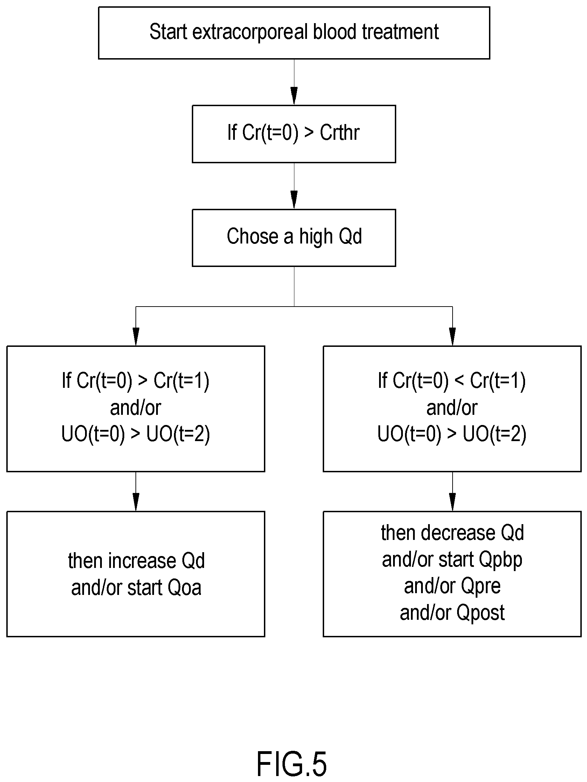

Referring to figure 5, if Cr(t=0) is above the creatinine threshold "$Cr_{thr}$" indicating Chronic Kidney Disease (CKD), then chose a high diuretic flow rate "$Q_d$" and then:

if Cr(t=0) > Cr(t=1) and/or Uo(t=0) > UO(t=1), then increase the diuretic flow rate "$Q_d$" and/or give osmotic agent (especially if blood pressure "BP" is low);

if Cr(t=0) < Cr(t=1) and U0(t=0) > UO(t=1), then decrease the diuretic flow rate "$Q_d$" and/or give infusion fluid (especially if blood pressure "BP" is low).

If the above outlined situation of creatinine is combined with Htc (t=0) < Htc (t=1) (indicating low plasma concentration and that an extra accumulated fluid is not in the vessels but outside), then add glucose or a buffer solution or saline depending on other underlying problems the patient may have.

If Htc (t=0) < Htc (t=1), then decrease the diuretic flow rate "$Q_d$", or, if UO (t=0) < UO (t=1), then give infusion fluid and check Htc and UO again after some time (e.g. 1 hour). If after such time the urine output "UO" is still increasing and the hematocrit "Htc" is getting better (i.e. decreasing), then continue with therapy as suggested and continue infusion of infusion fluid.

If the urine output "UO" increases, then one may increase the diuretic flow rate "$Q_d$" and adapt for the goal being reached and filtration flow rate "$Q_{fil}$" may be decreased, since it is always better to remove fluid through natural means.

If the access pressure "$P_{acc}$" is high ($P_{acc}$ (t=0) > $P_{acc\,high}$) and the hematocrit is high (Htc > $HtC_{thr}$) and the return pressure "$P_{ret}$" is low ($P_{ret}$ < $P_{ret\,low}$), then check the access for possible clot formation.

If the access pressure "$P_{acc}$" is above the high access pressure threshold "$P_{acc\,high}$" or within the access pressure range and the blood pressure "BP" is greater than 100 mmHg systolic and UO (t=0) < UO (t=1) or, during the treatment, the patient shows clinical signs of fluid overload, such as shortness of breath etc., then administering a bolus (e.g. 40 mg to 80 mg) of diuretic (e.g. furosemide) and checking again blood pressure and the effect of such administration.

If heart rate "HR" starts increasing systematically and at the same time blood pressure "BP" decreases slightly, then a routine for continuous check of blood pressure is triggered or the blood flow rate "$Q_b$" and/or the diuretic flow rate "$Q_d$" is/are reduced. Then, if the blood pressure "BP" becomes stable these changes are maintained, otherwise, if the blood pressure "BP" increases, then the initial settings with higher diuretic flow rate "$Q_d$" and/or blood flow rate "$Q_b$" are recovered.

If BP < 100 mmHg systolic, then give osmotic agent (e.g. albumin) and check blood pressure "BP". If blood pressure "BP" increases, then maintain the diuretic flow rate "$Q_d$". If blood pressure "BP" is still low, then decrease the diuretic flow rate "$Q_d$" and/or the blood flow rate "$Q_b$" and/or give infusion fluid to increase the blood pressure "BP".

If the urine output "UO" is below a urine threshold and the blood pressure "BP" is within the blood pressure range, then increase the effluent flow rate "$Q_{eff}$" and the filtration flow rate "$Q_{fil}$" and maintain the diuretic flow rate "$Q_d$".

If the urine output "UO" is not increasing during treatment but the blood pressure "BP" is high and the access pressure "$P_{acc}$" is high, then increase the blood flow rate "$Q_b$" and/or the diuretic flow rate "$Q_d$".

If patient "P" is taking antihypertensive medication, those are to be paused during the treatment.

The script or algorithm in the control unit 100 may also check a difference between the access pressure "$P_{acc}$" and the return pressure "$P_{ret}$" during the treatment and look at the results. If the pressure difference ($P_{acc}$ - $P_{ret}$) is getting bigger, depending on which way, it can indicate either good effect of diuretics (if fluid is added to blood from extra/intracellular liquid) or unfavorable changes (if fluid is only removed from blood and not from extra/intracellular liquid and this may cause damage to kidneys). The script or algorithm may also check the effect of osmotic agent (e.g. albumin) and the time it takes to achieve good results.

The script or algorithm in the control unit 100 may also control the effluent flow rate "$Q_{eff}$", the dialysis flow rate "$Q_{dial}$", the filtration flow rate "$Q_{fil}$" and the patient fluid removal rate "$Q_{pfr}$" and make sure that the changes in the diuretic flow rate "$Q_d$" and the osmotic agent flow rate "$Q_{oa}$" will be favorable (i.e. reduced along the treatment). If not, it will ask for e.g. creatinine "Cr" to make sure that the kidney have not worsened and/or calculate the post-filter hematocrit "$Htc_{pd}$" to make sure that the blood has not been concentrated too much. For instance, the post-filter hematocrit "$Htc_{pd}$" may be calculated through the following formula:

$$Htc_{pd} = Q_b \times 60 \times (Htc/(Q_b \times 60 + Q_{pbp} + Q_{pre} - Q_{fil}))$$

Then, the filtration flow rate "$Q_{fil}$" may be continued to be increased as long as hematocrit "Htc" and creatinine "Cr" have not increased too much.

The control unit 100 may also command the apparatus 1 to send an alarm signal if the access pressure is below a low alarm access pressure threshold or above a high alarm access pressure threshold.

## Claims

1.   An apparatus for extracorporeal blood treatment, comprising:

   a treatment unit (2);
   a blood circuit coupled to the treatment unit (2) and comprising a blood removal line (6) and a blood return line (7) connectable to a vascular system of a patient (P);
   a blood pump (10) configured to be coupled to a pump section of the blood circuit to deliver a blood flow rate ($Q_b$);
   an effluent line (12) connected to the treatment unit (2);
   an effluent pump (13) configured to be coupled to a pump section of the effluent line (12) to deliver an effluent flow rate ($Q_{eff}$); and
   a control unit (100) connected at least to the blood pump (10) and configured for receiving at least one input patient parameter and/or at least one input apparatus parameter;
   **characterized in that** the apparatus further comprises:

   a source (26) of at least one diuretic coupled to a diuretic pump (27) and connected to the blood circuit or connectable to the vascular system of the patient (P), wherein the diuretic pump (27) is configured to deliver a diuretic flow rate ($Q_d$);
   a source (29) of at least one osmotic agent coupled to an osmotic agent pump (30) and connected to the blood circuit or connectable to the vascular system of the patient (P),
   wherein the osmotic agent pump (30) is configured to deliver an osmotic agent flow rate ($Q_{oa}$);
   wherein the control unit (100) is connected to the diuretic pump (27) and the osmotic agent pump (30);
   wherein, during an extracorporeal blood treatment, the control unit (100) is programmed for driving at least the diuretic pump (27) and/or the osmotic agent pump (30) as a function of said at least one input patient parameter and/or as a function of at least one input apparatus parameter.

2.   The apparatus of claim 1, comprising or being connected to at least one sensor connected to the control unit (100), wherein the at least one sensor is configured to detect measured values of the at least one input patient parameter and/or the at least one input apparatus parameter, and wherein the control unit (100) is configured for monitoring said measured values over time and for driving at least the diuretic pump (27) and/or the osmotic agent pump (30) as a function of variation over time of said measured values.

3.   The apparatus of claim 1 or 2, wherein the control unit (100) is configured for receiving a prescription value of the at least one input patient parameter and/or the at least one input apparatus parameter and for driving at least the diuretic pump (27) and/or the osmotic agent pump (30) as a function of the prescription value of the input patient parameter and/or the input apparatus parameter, optionally as a function of a comparison between said prescription value with measured values of the input patient parameter and/or the input apparatus parameter.

4.   The apparatus of any of claims 2 to 3, wherein the at least one sensor is configured to detect the measured values of at least one of the following input patient parameters: heart rate (HR), blood pressure (BP), urine output (UO), consciousness level, body weight (BW), creatinine (Cr), hematocrit (Htc), level of electrolytes; wherein the control unit (100) is configured for receiving a prescription value of the at least one of the following input apparatus parameter: blood flow rate ($Q_b$), effluent flow rate ($Q_{eff}$), filtration flow rate ($Q_{fil}$); wherein the at least one sensor is configured to detect the measured values of at least one of the following input apparatus parameters: blood flow rate ($Q_b$), effluent flow rate ($Q_{eff}$), filtration flow rate ($Q_{fil}$), access pressure ($P_{acc}$), return pressure ($P_{ret}$).

5. The apparatus of claim 4, further comprising:

at least one infusion line (16, 17, 18) connected to the blood circuit and to a source (19, 21 23) of at least one infusion fluid;
an infusion pump (20, 22, 24) configured to be coupled to a pump section of said at least one infusion line (16, 17, 18) and to deliver an infusion flow rate ($Q_{pbp}$, $Q_{pre}$, $Q_{post}$);
a dialysis line (11) connected to the treatment unit (2) and to a source (14) of a dialysis fluid; a dialysis pump (15) configured to be coupled to a pump section of the dialysis line (11) and to deliver a dialysis flow rate ($Q_{dial}$);
wherein the control unit (100) is configured for receiving the prescription value of the at least one of the following input apparatus parameter: infusion flow rate ($Q_{pbp}$, $Q_{pre}$, $Q_{post}$), dialysis flow rate ($Q_{dial}$) and/or optionally wherein the at least one sensor is configured to detect the measured values of at least one of the following input apparatus parameters: infusion flow rate ($Q_{inf}$), dialysis flow rate ($Q_{dial}$).

6. The apparatus of claim 4 or 5, wherein the control unit (100) is configured for performing the following procedure:

- if, at the start of the extracorporeal blood treatment, the access pressure ($P_{acc}$) is above a high access pressure threshold and the blood pressure (BP) is above a blood pressure threshold or within an access pressure range, then commanding the blood pump (10) to increase the blood flow rate ($Q_b$) and/or commanding the effluent pump (13) to increase the effluent flow rate ($Q_{eff}$) and the filtration flow rate ($Q_{fil}$) and/or commanding the diuretic pump (27) to start or increase the diuretic flow rate ($Q_d$);
- if, at the start of the extracorporeal blood treatment, the access pressure ($P_{acc}$) is below a low access pressure threshold and the blood pressure (BP) is below a blood pressure threshold, then commanding the blood pump (10) to decrease the blood flow rate ($Q_b$) and/or commanding the effluent pump (13) to decrease the effluent flow rate ($Q_{eff}$) and the filtration flow rate ($Q_{fil}$) and/or commanding the osmotic agent pump (30) to start or increase the osmotic agent ($Q_{oa}$) and/or commanding the diuretic pump (27) to start or increase the diuretic flow rate ($Q_d$).

7. The apparatus of claim 6, wherein the control unit (100) is configured for performing the following procedure:

i. if the access pressure ($P_{acc}$) decreases over time, then starting the diuretic pump (27) and/or the osmotic agent pump (30) to deliver the diuretic flow rate ($Q_d$) and/or the osmotic agent flow rate ($Q_{oa}$); or
ii. if the access pressure ($P_{acc}$) increases over time, taking no action or commanding the effluent pump (13) to increase the effluent flow rate ($Q_{eff}$) and the filtration flow rate ($Q_{fil}$).

8. The apparatus of any of claims 4, 5, 6, 7, wherein, if at the start of the extracorporeal blood treatment the creatinine (Cr) is above a creatinine threshold indicating Chronic Kidney Disease, the control unit (100) is configured for commanding the diuretic pump (27) to increase the diuretic flow rate ($Q_d$).

9. The apparatus of claim 8 when depending on claim 5, wherein the control unit (100) is configured for performing the following procedure:

- if the creatinine (Cr) starts decreasing over time and/or the urine output (UO) starts decreasing over time, then commanding the diuretic pump (27) to increase the diuretic flow rate ($Q_d$) and/or commanding the osmotic pump (30) to start;
- if the creatinine (Cr) starts increasing over time and/or the urine output (UO) starts decreasing over time, then commanding the diuretic pump (27) to decrease the diuretic flow rate ($Q_d$) and/or commanding the infusion pump (20, 22, 24) to start the infusion flow rate ($Q_{pbp}$, $Q_{pre}$, $Q_{post}$).

10. The apparatus of any of claims 4, 5, 6, 7, 8, 9, wherein the control unit (100) is configured for performing the following procedure:

- if hematocrit (Htc) increases over time, then commanding the diuretic pump (27) to decrease the diuretic flow rate ($Q_d$); or
- if the urine output (UO) increases over time, then commanding the infusion pump (20, 22, 24) to start or to increase the infusion flow rate ($Q_{pbp}$, $Q_{pre}$, $Q_{post}$); then
- if the urine output (UO) still increases over time and hematocrit (Htc) starts decreasing, then maintain the infusion flow rate ($Q_{pbp}$, $Q_{pre}$, $Q_{post}$).

11. The apparatus of any of claims 1 to 10, wherein the control unit (100) is configured for receiving data of the diuretic and/or data of the osmotic agent, and wherein the osmotic agent comprises albumin or amino acids or mannitol or hypertonic saline or glucose solution; wherein the diuretic comprises furosemide or torsemide or bumetanide or ethacrynic acid or metolazone or chlorthalidone or hydrochlorothiazide or chlorothiazide or spironolactone or eplererone.

12. The apparatus of any of claims 1 to 11, wherein a diuretic line (25) is connected to the source (29) of the at least one diuretic and to the patient (P) or to the blood circuit, the diuretic pump (27) being configured to be coupled to a pump section of the diuretic line (29) to deliver the diuretic flow rate ($Q_d$); wherein an osmotic agent line (28) is connected to the source (29) of the at least one osmotic agent and to the patient (P) or to the blood circuit, the osmotic agent pump (30) being configured to be coupled to a pump section of the osmotic agent line (28) to deliver the osmotic agent flow rate ($Q_{oa}$); wherein the apparatus (1) comprises an integrated disposable set, wherein the treatment unit (2), the blood circuit, the effluent line (12), the diuretic line (25) and/or the osmotic agent line (28) are grouped together and connected in the integrated disposable set,
wherein said apparatus is a continuous renal replacement therapy (CRRT) system for intensive care treatments.

13. The apparatus of any of claims 1 to 12, further comprising:

at least one auxiliary line connected to the blood circuit and to a source of at least one compensation substance, such as potassium or bicarbonate;
a pump or syringe configured to be coupled to a pump section of said at least one auxiliary line and to deliver a flow rate of the compensation substance;
wherein the at least one input apparatus parameter further comprises the flow rate of the compensation substance.

14. The apparatus of claim 7, wherein, if situation i. occurs, the control unit (100) is configured for performing the following procedure:

- if the access pressure keeps on decreasing over time, then starting the osmotic agent pump (30) and/or the diuretic pump (27) or commanding the diuretic pump (27) to increase the diuretic flow rate ($Q_d$) and/or commanding the osmotic agent pump (30) to increase the osmotic agent flow rate ($Q_{oa}$); or
- if the access pressure starts increasing over time, then commanding the diuretic pump (27) to maintain the diuretic flow rate ($Q_d$) and/or commanding the osmotic agent pump (30) to maintain the osmotic agent flow rate ($Q_{oa}$).

15. The apparatus of claim 4, wherein:

if the blood pressure is within a blood pressure range, the control unit (100) is configured for commanding the blood pump (10) to increase the blood flow rate ($Q_b$), commanding the diuretic pump (27) to increase the diuretic flow rate ($Q_d$) and commanding the effluent pump (13) to increase the effluent flow rate ($Q_{eff}$) and the filtration flow rate ($Q_{fil}$);
if the blood pressure is below a blood pressure threshold, the control unit (100) is configured for starting the osmotic agent pump (30) or commanding the osmotic agent pump (27) to increase the osmotic agent flow rate ($Q_{oa}$) till the blood pressure is stabilized and then starting the diuretic pump (30) or commanding the diuretic pump (30) to increase the diuretic flow rate ($Q_d$).

**Patentansprüche**

1. Vorrichtung zur extrakorporalen Blutbehandlung, umfassend:

eine Behandlungseinheit (2);
einen Blutkreislauf, der mit der Behandlungseinheit (2) gekoppelt ist und eine Blutentnahmeleitung (6) und eine Blutrückführungsleitung (7) umfasst, die mit einem Gefäßsystem eines Patienten (P) verbindbar sind;
eine Blutpumpe (10), die dazu ausgelegt ist, mit einem Pumpenabschnitt des Blutkreislaufs gekoppelt zu werden, um eine Blutflussrate ($Q_b$) zu liefern;
eine Abwasserleitung (12), die mit der Behandlungseinheit (2) verbunden ist;
eine Abwasserpumpe (13), die dazu ausgelegt ist, mit einem Pumpenabschnitt der Abwasserleitung (12) gekoppelt zu werden, um eine Abwasserdurchflussrate ($Q_{eff}$) zu liefern; und

eine Steuereinheit (100), die mindestens mit der Blutpumpe (10) verbunden und zum Empfangen von mindestens einem Eingabepatientenparameter und/oder mindestens einem Eingabevorrichtungsparameter ausgelegt ist;

**dadurch gekennzeichnet, dass** die Vorrichtung ferner umfasst:

eine Quelle (26) von mindestens einem Diuretikum, die mit einer Diuretikumpumpe (27) gekoppelt und mit dem Blutkreislauf verbunden oder mit dem Gefäßsystem des Patienten (P) verbindbar ist, wobei die Diuretikumpumpe (27) zum Liefern einer Diuretikumdurchflussrate ($Q_d$) ausgelegt ist;

eine Quelle (29) für mindestens ein osmotisches Mittel, die mit einer Pumpe (30) für das osmotische Mittel gekoppelt und mit dem Blutkreislauf verbunden oder mit dem Gefäßsystem des Patienten (P) verbindbar ist, wobei die Pumpe (30) für das osmotische Mittel zum Liefern einer Durchflussrate ($Q_{oa}$) des osmotischen Mittels ausgelegt ist;

wobei die Steuereinheit (100) mit der Diuretikumpumpe (27) und der Pumpe (30) für das osmotische Mittel verbunden ist;

wobei während einer extrakorporalen Blutbehandlung die Steuereinheit (100) zum Antreiben mindestens der Diuretikumpumpe (27) und/oder der Pumpe (30) für das osmotische Mittel in Abhängigkeit von dem mindestens einen Eingabepatientenparameter und/oder in Abhängigkeit von mindestens einem Eingabevorrichtungsparameter ausgelegt ist.

2. Vorrichtung nach Anspruch 1, umfassend oder verbunden mit mindestens einem Sensor, der mit der Steuereinheit (100) verbunden ist, wobei der mindestens eine Sensor zum Erfassen von Messwerten des mindestens einen Eingabepatientenparameters und/oder des mindestens einen Eingabevorrichtungsparameters ausgelegt ist, und wobei die Steuereinheit (100) zum Überwachen dieser Messwerte über die Zeit und zum Antreiben mindestens der Diuretikumpumpe (27) und/oder der Pumpe (30) für das osmotische Mittel in Abhängigkeit von einer zeitlichen Variation der Messwerte ausgelegt ist.

3. Vorrichtung nach Anspruch 1 oder 2, wobei die Steuereinheit (100) zum Empfangen eines Verschreibungswerts des mindestens einen Eingabepatientenparameters und/oder des mindestens einen Eingabevorrichtungsparameters und zum Antreiben mindestens der Diuretikumpumpe (27) und/oder der Pumpe (30) für das osmotische Mittel in Abhängigkeit von dem Verschreibungswert des Eingabepatientenparameters und/oder des Eingabevorrichtungsparameters ausgelegt ist, optional in Abhängigkeit von einem Vergleich zwischen dem Verschreibungswert mit den Messwerten des Eingabepatientenparameters und/oder des Eingabevorrichtungsparameters.

4. Vorrichtung nach einem der Ansprüche 2 bis 3, wobei der mindestens eine Sensor zum Erfassen der Messwerte von mindestens einem der folgenden Eingabepatientenparameter ausgelegt ist: Herzfrequenz (HR), Blutdruck (BP), Urinausscheidung (UO), Bewusstseinsniveau, Körpergewicht (BW), Kreatinin (Cr), Hämatokrit (Htc), Elektrolytspiegel; wobei die Steuereinheit (100) zum Empfangen eines Verschreibungswerts des mindestens einen der folgenden Eingabevorrichtungsparameter ausgelegt ist: Blutflussrate ($Q_b$), Abwasserdurchflussrate ($G_{eff}$), Filtrationsdurchflussrate ($Q_{fil}$);

wobei der mindestens eine Sensor zum Erfassen der Messwerte von mindestens einem der folgenden Eingabevorrichtungsparameter ausgelegt ist: Blutflussrate ($Q_b$), Abwasserdurchflussrate ($Q_{eff}$), Filtrationsdurchflussrate ($Q_{fil}$), Zugangsdruck ($P_{acc}$), Rücklaufdruck ($P_{ret}$).

5. Vorrichtung nach Anspruch 4, ferner umfassend:

mindestens eine Infusionsleitung (16, 17, 18), die mit dem Blutkreislauf und mit einer Quelle (19, 21, 23) für mindestens eine Infusionsflüssigkeit verbunden ist;

eine Infusionspumpe (20, 22, 24), die dazu ausgelegt ist, mit einem Pumpenabschnitt der mindestens einen Infusionsleitung (16, 17, 18) gekoppelt zu werden und eine Infusionsdurchflussrate ($Q_{pbp}$, $Q_{pre}$, $Q_{post}$) zu liefern;

eine Dialyseleitung (11), die mit der Behandlungseinheit (2) und mit einer Quelle (14) für eine Dialyseflüssigkeit verbunden ist;

eine Dialysepumpe (15), die dazu ausgelegt ist, mit einem Pumpenabschnitt der Dialyseleitung (11) gekoppelt zu werden und eine Dialysedurchflussrate ($Q_{dial}$) zu liefern;

wobei die Steuereinheit (100) zum Empfangen des Verschreibungswerts des mindestens einen der folgenden Eingabevorrichtungsparameter ausgelegt ist: Infusionsdurchflussrate ($Q_{pbp}$, $Q_{pre}$, $Q_{post}$), Dialysedurchflussrate ($Q_{dial}$) und/oder wobei optional der mindestens eine Sensor zum Erfassen der Messwerte mindestens eines der folgenden Eingabevorrichtungsparameter ausgelegt ist: Infusionsdurchflussrate ($Q_{inf}$), Dialysedurchflussrate

($Q_{dial}$).

6. Vorrichtung nach Anspruch 4 oder 5, wobei die Steuereinheit (100) zum Durchführen des folgenden Verfahrens ausgelegt ist:

- wenn zu Beginn der extrakorporalen Blutbehandlung der Zugangsdruck ($P_{acc}$) über einem hohen Zugangsdruckschwellenwert liegt und der Blutdruck (BP) über einem Blutdruckschwellenwert oder innerhalb eines Zugangsdruckbereichs liegt, dann Erteilen eines Befehls an die Blutpumpe (10), die Blutflussrate ($Q_b$) zu erhöhen und/oder Erteilen eines Befehls an die Abwasserpumpe (13), die Abwasserdurchflussrate ($Q_{eff}$) und die Filtrationsdurchflussrate ($Q_{fil}$) zu erhöhen und/oder Erteilen eines Befehls an die Diuretikumpumpe (27), die Diuretikumdurchflussrate ($Q_d$) zu starten oder zu erhöhen;
- wenn zu Beginn der extrakorporalen Blutbehandlung der Zugangsdruck ($P_{acc}$) unter einem niedrigen Zugangsdruckschwellenwert liegt und der Blutdruck (BP) unter einem Blutdruckschwellenwert liegt, dann Erteilen eines Befehls an die Blutpumpe (10), die Blutflussrate ($Q_b$) zu verringern und/oder Erteilen eines Befehls an die Abwasserpumpe (13), die Abwasserdurchflussrate ($Q_{eff}$) und die Filtrationsdurchflussrate ($Q_{fil}$) zu verringern und/oder Erteilen eines Befehls an die Pumpe (30) für das osmotische Mittel, das osmotische Mittel ($Q_{oa}$) zu starten oder zu erhöhen, und/oder Erteilens eines Befehls an die Diuretikumpumpe (27), die Diuretikumdurchflussrate ($Q_d$) zu starten oder zu erhöhen.

7. Vorrichtung nach Anspruch 6, wobei die Steuereinheit (100) zum Durchführen des folgenden Verfahrens ausgelegt ist:

i. wenn der Zugangsdruck ($P_{acc}$) mit der Zeit abnimmt, dann Starten der Diuretikumpumpe (27) und/oder der Pumpe (30) für das osmotische Mittel, um die Diuretikumdurchflussrate ($Q_d$) und/oder die Durchflussrate des osmotischen Mittels ($Q_{oa}$) zu liefern; oder
ii. wenn der Zugangsdruck ($P_{acc}$) mit der Zeit zunimmt, kein Ergreifen von Maßnahmen oder Erteilen eines Befehls an die Abwasserpumpe (13), die Abwasserdurchflussrate ($Q_{eff}$) und die Filtrationsdurchflussrate ($Q_{fil}$) zu erhöhen.

8. Vorrichtung nach einem der Ansprüche 4, 5, 6, 7, wobei, wenn zu Beginn der extrakorporalen Blutbehandlung das Kreatinin (Cr) über einem Kreatininschwellenwert liegt, der auf eine chronische Nierenerkrankung hinweist, die Steuereinheit (100) dazu ausgelegt ist, der Diuretikumpumpe (27) den Befehl zu erteilen, die Diuretikumdurchflussrate ($Q_d$) zu erhöhen.

9. Vorrichtung nach Anspruch 8, in Abhängigkeit von Anspruch 5, wobei die Steuereinheit (100) zum Durchführen des folgenden Verfahrens ausgelegt ist:

- wenn das Kreatinin (Cr) mit der Zeit beginnt abzunehmen und/oder die Urinausscheidung (UO) mit der Zeit beginnt abzunehmen, dann Erteilen eines Befehls an die Diuretikumpumpe (27), die Diuretikumdurchflussrate ($Q_d$) zu erhöhen und/oder Erteilen eines Befehls an die osmotische Pumpe (30) zu starten;
- wenn das Kreatinin (Cr) mit der Zeit beginnt zuzunehmen und/oder die Urinausscheidung (UO) mit der Zeit beginnt zuzunehmen, dann Erteilen eines Befehls an die Diuretikumpumpe (27), die Diuretikumdurchflussrate ($Q_d$) zu verringern und/oder Erteilen eines Befehls an die Infusionspumpe (20, 22, 24), die Infusionsdurchflussrate ($Q_{pbp}$, $Q_{pre}$, $Q_{post}$) zu starten.

10. Vorrichtung nach einem der Ansprüche 4, 5, 6, 7, 8, 9, wobei die Steuereinheit (100) zum Durchführen des folgenden Verfahrens ausgelegt ist:

- wenn der Hämatokrit (Htc) mit der Zeit zunimmt, dann Erteilen eines Befehls an die Diuretikumpumpe (27), die Diuretikumdurchflussrate (Qd) zu verringern; oder
- wenn die Urinausscheidung (UO) mit der Zeit zunimmt, dann Erteilen eines Befehls an die Infusionspumpe (20, 22, 24), die Infusionsdurchflussrate ($Q_{pbp}$, $Q_{pre}$, $Q_{post}$) zu starten oder zu erhöhen; dann
- wenn die Urinausscheidung (UO) mit der Zeit weiter zunimmt und der Hämatokrit (Htc) beginnt abzunehmen, dann Aufrechterhalten der Infusionsdurchflussrate ($Q_{pbp}$, $Q_{pre}$, $Q_{post}$).

11. Vorrichtung nach einem der Ansprüche 1 bis 10, wobei die Steuereinheit (100) zum Empfangen von Daten des Diuretikums und/oder Daten des osmotischen Mittels ausgelegt ist, und wobei das osmotische Mittel Albumin oder Aminosäuren oder Mannitol oder hypertone Kochsalzlösung oder Glukoselösung umfasst;

wobei das Diuretikum Furosemid oder Torsemid oder Bumetanid oder Ethacrynsäure oder Metolazon oder Chlorthalidon oder Hydrochlorothiazid oder Chlorothiazid oder Spironolacton oder Eplereron umfasst.

12. Vorrichtung nach einem der Ansprüche 1 bis 11, wobei eine Diuretikumleitung (25) mit der Quelle (29) des mindestens einen Diuretikums und mit dem Patienten (P) oder mit dem Blutkreislauf verbunden ist, wobei die Diuretikumpumpe (27) zur Kopplung mit einem Pumpenabschnitt der Diuretikumleitung (29) ausgelegt ist, um die Diuretikumdurchflussrate ($Q_d$) zu liefern; wobei eine Leitung (28) für ein osmotisches Mittel mit der Quelle (29) des mindestens einen osmotischen Mittels und mit dem Patienten (P) oder mit dem Blutkreislauf verbunden ist, wobei die Pumpe (30) für das osmotische Mittel zur Kopplung mit einem Pumpenabschnitt der Leitung (28) für das osmotische Mittel ausgelegt ist, um die Durchflussrate ($Q_{oa}$) des osmotischen Mittels zu liefern; wobei die Vorrichtung (1) ein integriertes Einweg-Set umfasst, wobei die Behandlungseinheit (2), der Blutkreislauf, die Abwasserleitung (12), die Diuretikumleitung (25) und/oder die Leitung (28) für das osmotische Mittel in dem integrierten Einweg-Set gruppiert und verbunden sind, wobei die Vorrichtung ein kontinuierliches Nierenersatztherapiesystem (CRRT) für intensivmedizinische Behandlungen ist.

13. Vorrichtung nach einem der Ansprüche 1 bis 12, ferner umfassend:

mindestens eine Hilfsleitung, die mit dem Blutkreislauf und mit einer Quelle für mindestens eine Kompensationssubstanz, wie Kalium oder Bicarbonat, verbunden ist;
eine Pumpe oder Spritze, die zur Kopplung mit einem Pumpenabschnitt der mindestens einen Hilfsleitung und zum Liefern einer Durchflussrate der Kompensationssubstanz ausgelegt ist;
wobei der mindestens eine Eingabevorrichtungsparameter ferner die Durchflussrate der Kompensationssubstanz umfasst.

14. Vorrichtung nach Anspruch 7, wobei die Steuereinheit (100) für den Fall des Eintretens von Situation i. zum Durchführen des folgenden Verfahrens ausgelegt ist:

- wenn der Zugangsdruck mit der Zeit weiter abnimmt, dann Starten der Pumpe (30) für das osmotische Mittel und/oder der Diuretikumpumpe (27) oder Erteilen eines Befehls an die Diuretikumpumpe (27), die Diuretikumdurchflussrate ($Q_d$) zu erhöhen und/oder Erteilen eines Befehls an die Pumpe (30) für das osmotische Mittel, die Durchflussrate des osmotischen Mittels (Qoa) zu erhöhen; oder
- wenn der Zugangsdruck mit der Zeit zunimmt, dann Erteilen eines Befehls an die Diuretikumpumpe (27), die Diuretikumdurchflussrate ($Q_d$) aufrechtzuerhalten und/oder Erteilen eines Befehls an die Pumpe (30) für das osmotische Mittel, die Durchflussrate des osmotischen Mittels ($Q_{oa}$) aufrechtzuerhalten.

15. Vorrichtung nach Anspruch 4, wobei:

wenn der Blutdruck innerhalb eines Blutdruckbereichs liegt, die Steuereinheit (100) dazu ausgelegt ist, der Blutpumpe (10) den Befehl zu erteilen, die Blutflussrate ($Q_b$) zu erhöhen, der Diuretikumpumpe (27) den Befehl zu erteilen, die Diuretikumdurchflussrate ($Q_d$) zu erhöhen, und der Abwasserpumpe (13) den Befehl zu erteilen, die Abwasserdurchflussrate ($Q_{eff}$) und die Filtrationsdurchflussrate ($Q_{fil}$) zu erhöhen;
wenn der Blutdruck unter einem Blutdruckschwellenwert liegt, die Steuereinheit (100) dazu ausgelegt ist, die Pumpe (30) für das osmotische Mittel zu starten oder der Pumpe (27) für das osmotische Mittel den Befehl zu erteilen, die Durchflussrate des osmotischen Mittels ($Q_{oa}$) zu erhöhen, bis der Blutdruck stabilisiert ist, und dann die Diuretikumpumpe (30) zu starten oder der Diuretikumpumpe (30) den Befehl zu erteilen, die Diuretikumdurchflussrate (Qd) zu erhöhen.

## Revendications

1. Appareil de traitement extracorporel du sang, comprenant :

une unité de traitement (2) ;
un circuit sanguin couplé à l'unité de traitement (2) et comprenant une ligne d'évacuation du sang (6) et une ligne de retour du sang (7) pouvant être raccordées au système vasculaire d'un patient (P) ;
une pompe à sang (10) configurée pour être couplée à une section de pompe du circuit sanguin afin de fournir un débit sanguin ($Q_b$) ;
une ligne d'effluent (12) reliée à l'unité de traitement (2) ;

une pompe à effluent (13) configurée pour être couplée à une section de pompe de la ligne d'effluent (12) afin de fournir un débit d'effluent ($Q_{eff}$) ; et

une unité de commande (100) connectée au moins à la pompe à sang (10) et configurée pour recevoir au moins un paramètre d'entrée du patient et/ou au moins un paramètre d'entrée de l'appareil ;

**caractérisé en ce que** l'appareil comprend en outre :

une source (26) d'au moins un diurétique couplée à une pompe à diurétique (27) et connectée au circuit sanguin ou connectable au système vasculaire du patient (P), où la pompe à diurétique (27) est configurée pour délivrer un débit de diurétique ($Q_d$) ;

une source (29) d'au moins un agent osmotique couplée à une pompe à agent osmotique (30) et connectée au circuit sanguin ou pouvant être connectée au système vasculaire du patient (P), où la pompe à agent osmotique (30) est configurée pour délivrer un débit d'agent osmotique ($Q_{oa}$) ;

où l'unité de commande (100) est connectée à la pompe à diurétique (27) et à la pompe à agent osmotique (30) ;

où, pendant un traitement extracorporel du sang, l'unité de commande (100) est programmée pour commander au moins la pompe à diurétique (27) et/ou la pompe à agent osmotique (30) en fonction dudit au moins un paramètre de patient d'entrée et/ou en fonction d'au moins un paramètre de l'appareil d'entrée.

2. Appareil selon la revendication 1, comprenant ou étant connecté à au moins un capteur connecté à l'unité de commande (100), où l'au moins un capteur est configuré pour détecter des valeurs mesurées de l'au moins un paramètre de patient d'entrée et/ou de l'au moins un paramètre d'appareil d'entrée, et où l'unité de commande (100) est configurée pour surveiller lesdites valeurs mesurées dans le temps et pour commander au moins la pompe à diurétique (27) et/ou la pompe à agent osmotique (30) en fonction de la variation dans le temps desdites valeurs mesurées.

3. Appareil selon la revendication 1 ou la revendication 2, dans lequel l'unité de commande (100) est configurée pour recevoir une valeur de prescription de l'au moins un paramètre de patient d'entrée et/ou de l'au moins un paramètre d'appareil d'entrée et pour commander au moins la pompe à diurétique (27) et/ou la pompe à agent osmotique (30) en fonction de la valeur de prescription du paramètre de patient d'entrée et/ou du paramètre d'appareil d'entrée, éventuellement en fonction d'une comparaison entre ladite valeur de prescription et des valeurs mesurées du paramètre de patient d'entrée et/ou du paramètre de l'appareil d'entrée.

4. Appareil selon l'une quelconque des revendications 2 et 3, dans lequel l'au moins un capteur est configuré pour détecter les valeurs mesurées d'au moins l'un des paramètres de patient d'entrée suivants : la fréquence cardiaque (HR), la pression artérielle (BP), le débit urinaire (UO), le niveau de conscience, le poids corporel (BW), la créatinine (Cr), l'hématocrite (Htc), le niveau d'électrolytes ; où l'unité de commande (100) est configurée pour recevoir une valeur de prescription d'au moins un des paramètres d'entrée de l'appareil suivants :

le débit sanguin ($Q_b$), le débit d'effluent ($Q_{eff}$), le débit de filtration ($Q_{fil}$) ;

où l'au moins un capteur est configuré pour détecter les valeurs mesurées d'au moins un des paramètres d'entrée de l'appareil suivants : le débit sanguin ($Q_b$), le débit d'effluent ($Q_{eff}$), le débit de filtration ($Q_{fil}$), la pression d'accès ($P_{acc}$), la pression de retour ($P_{ret}$).

5. Appareil selon la revendication 4, comprenant en outre :

au moins une ligne de perfusion (16, 17, 18) connectée au circuit sanguin et à une source (19, 21, 23) d'au moins un fluide de perfusion ;

une pompe de perfusion (20, 22, 24) configurée pour être couplée à une section de pompe de ladite au moins une ligne de perfusion (16, 17, 18) et pour délivrer un débit de perfusion ($Q_{pbp}$, $Q_{pre}$, $Q_{post}$) ;

une ligne de dialyse (11) reliée à l'unité de traitement (2) et à une source (14) d'un fluide de dialyse ;

une pompe de dialyse (15) configurée pour être couplée à une section de pompe de la ligne de dialyse (11) et pour délivrer un débit de dialyse ($Q_{dial}$) ;

où l'unité de commande (100) est configurée pour recevoir la valeur de prescription d'au moins l'un des paramètres suivants de l'appareil d'entrée : le débit de perfusion ($Q_{pbp}$, $Q_{pre}$, $Q_{post}$), le débit de dialyse ($Q_{dial}$) et/ou, en option, dans lequel le ou les capteurs sont configurés pour détecter les valeurs mesurées d'au moins l'un des paramètres suivants de l'appareil d'entrée : le débit de perfusion ($Q_{inf}$), le débit de dialyse ($Q_{dial}$).

6. Appareil selon la revendication 4 ou la revendication 5, dans lequel l'unité de commande (100) est configurée pour exécuter la procédure suivante :

- si, au début du traitement extracorporel du sang, la pression d'accès ($P_{acc}$) est supérieure à un seuil de pression d'accès élevé et que la pression sanguine (BP) est supérieure à un seuil de pression sanguine ou se situe dans une plage de pression d'accès, commander à la pompe à sang (10) d'augmenter le débit sanguin ($Q_b$) et/ou commander à la pompe à effluent (13) d'augmenter le débit d'effluent ($Q_{eff}$) et le débit de filtration ($Q_{fil}$) et/ou commander à la pompe à diurétique (27) de démarrer ou d'augmenter le débit de diurétique ($Q_d$) ;
- si, au début du traitement extracorporel du sang, la pression d'accès ($P_{acc}$) est inférieure à un seuil de pression d'accès bas et que la pression sanguine (BP) est inférieure à un seuil de pression sanguine, commander à la pompe à sang (10) de diminuer le débit sanguin ($Q_b$) et/ou commander à la pompe à effluent (13) de diminuer le débit d'effluent ($Q_{eff}$) et le débit de filtration ($Q_{fil}$) et/ou commander à la pompe à agent osmotique (30) de démarrer ou d'augmenter l'agent osmotique ($Q_{oa}$) et/ou commander à la pompe à diurétique (27) de démarrer ou d'augmenter le débit de diurétique ($Q_d$).

7. Appareil selon la revendication 6, dans lequel l'unité de commande (100) est configurée pour exécuter la procédure suivante :

i. si la pression d'accès ($P_{acc}$) diminue au fil du temps, démarrer la pompe à diurétique (27) et/ou la pompe à agent osmotique (30) pour délivrer le débit de diurétique ($Q_d$) et/ou le débit d'agent osmotique ($Q_{oa}$) ; ou
ii. si la pression d'accès ($P_{acc}$) augmente au fil du temps, ne prendre aucune mesure ou commander à la pompe à effluent (13) d'augmenter le débit d'effluent ($Q_{eff}$) et le débit de filtration ($Q_{fil}$).

8. Appareil selon l'une quelconque des revendications 4, 5, 6, 7, dans lequel, si au début du traitement extracorporel du sang, la créatinine (Cr) est supérieure à un seuil de créatinine indiquant une maladie rénale chronique, l'unité de commande (100) est configurée pour commander à la pompe à diurétique (27) d'augmenter le débit de diurétique ($Q_d$).

9. Appareil selon la revendication 8, lorsqu'elle dépend de la revendication 5, dans lequel l'unité de commande (100) est configurée pour exécuter la procédure suivante :

- si la créatinine (Cr) commence à diminuer au fil du temps et/ou si le débit urinaire (UO) commence à diminuer au fil du temps, commander à la pompe à diurétique (27) d'augmenter le débit de diurétique ($Q_d$) et/ou commander à la pompe osmotique (30) de démarrer ;
- si la créatinine (Cr) commence à augmenter au fil du temps et/ou si le débit urinaire (UO) commence à diminuer au fil du temps, commander à la pompe à diurétique (27) de diminuer le débit de diurétique ($Q_d$) et/ou commander à la pompe de perfusion (20, 22, 24) de démarrer le débit de perfusion ($Q_{pbp}$, $Q_{pre}$, $Q_{post}$).

10. Appareil selon l'une quelconque des revendications 4, 5, 6, 7, 8, 9, dans lequel l'unité de commande (100) est configurée pour exécuter la procédure suivante :

- si l'hématocrite (Htc) augmente au fil du temps, commander à la pompe à diurétique (27) de diminuer le débit de diurétique ($Q_d$) ; ou
- si le débit urinaire (UO) augmente au fil du temps, commander à la pompe de perfusion (20, 22, 24) de démarrer ou d'augmenter le débit de perfusion ($Q_{pbp}$, $Q_{pre}$, $Q_{post}$) ; puis
- si le débit urinaire (UO) continue d'augmenter au fil du temps et que l'hématocrite (Htc) commence à diminuer, maintenir le débit de perfusion ($Q_{pbp}$, $Q_{pre}$, $Q_{post}$).

11. Appareil selon l'une quelconque des revendications 1 à 10, dans lequel l'unité de commande (100) est configurée pour recevoir des données relatives au diurétique et/ou des données relatives à l'agent osmotique, et dans lequel l'agent osmotique comprend de l'albumine ou des acides aminés ou du mannitol ou une solution saline hypertonique ou une solution de glucose ; dans lequel le diurétique comprend du furosémide ou du torsémide ou du bumétanide ou de l'acide éthacrynique ou de la métolazone ou de la chlorthalidone ou de l'hydrochlorothiazide ou de la chlorothiazide ou de la spironolactone ou de l'éplérérone.

12. Appareil selon l'une quelconque des revendications 1 à 11, dans lequel une ligne de diurétique (25) est reliée à la source (29) d'au moins un diurétique et au patient (P) ou au circuit sanguin, la pompe à diurétique (27) étant configurée pour être couplée à une section de pompe de la ligne de diurétique (29) afin de délivrer le débit de diurétique ($Q_d$) ; où une ligne d'agent osmotique (28) est reliée à la source (29) d'au moins un agent osmotique et au patient (P) ou au circuit sanguin, la pompe à agent osmotique (30) étant configurée pour être couplée à une section de pompe de la ligne d'agent osmotique (28) afin de délivrer le débit d'agent osmotique ($Q_{oa}$) ; où l'appareil (1) comprend un ensemble intégré jetable, où l'unité de traitement (2), le circuit sanguin, la ligne d'effluent (12), la ligne de diurétique (25) et/ou la ligne d'agent osmotique (28) sont regroupés et connectés dans l'ensemble intégré jetable, où ledit appareil est un système de thérapie de remplacement rénal continu (CRRT) pour des traitements de soins intensifs.

13. Appareil selon l'une quelconque des revendications 1 à 12, comprenant en outre :

au moins une ligne auxiliaire reliée au circuit sanguin et à une source d'au moins une substance de compensation, telle que du potassium ou du bicarbonate ;
une pompe ou une seringue configurée pour être couplée à une section de pompage de ladite au moins une ligne auxiliaire et pour délivrer un débit de la substance de compensation ;
où l'au moins un paramètre d'entrée de l'appareil comprend en outre le débit de la substance de compensation.

14. Appareil selon la revendication 7, dans lequel, si la situation i. se produit, l'unité de commande (100) est configurée pour exécuter la procédure suivante :

- si la pression d'accès continue de diminuer au fil du temps, démarrer la pompe à agent osmotique (30) et/ou la pompe à diurétique (27) ou commander à la pompe à diurétique (27) pour augmenter le débit diurétique ($Q_d$) et/ou commander à la pompe à agent osmotique (30) pour augmenter le débit d'agent osmotique ($Q_{oa}$) ; ou
- si la pression d'accès commence à augmenter au fil du temps, commander la pompe à diurétique (27) pour maintenir le débit de diurétique ($Q_d$) et/ou commander la pompe à agent osmotique (30) pour maintenir le débit d'agent osmotique ($Q_{oa}$).

15. Appareil selon la revendication 4, dans lequel :
si la pression sanguine se trouve dans une plage de pression sanguine, l'unité de commande (100) est configurée pour commander à la pompe à sang (10) d'augmenter le débit sanguin ($Q_b$), commander à la pompe à diurétique (27) d'augmenter le débit de diurétique ($Q_d$) et commander à la pompe à effluent (13) d'augmenter le débit d'effluent ($Q_{eff}$) et le débit de filtration ($Q_{fil}$) ; si la pression sanguine est inférieure à un seuil de pression sanguine, l'unité de commande (100) est configurée pour démarrer la pompe à agent osmotique (30) ou commander à la pompe à agent osmotique (27) d'augmenter le débit d'agent osmotique ($Q_{oa}$) jusqu'à ce que la pression sanguine se stabilise, puis démarrer la pompe à diurétique (30) ou commander à la pompe à diurétique (30) d'augmenter le débit de diurétique ($Q_d$).

FIG.1

EP 4 426 368 B1

FIG.2

EP 4 426 368 B1

EP 4 426 368 B1

```
┌─────────────────────────────────────────────┐
│      Start extracorporeal blood treatment     │
└─────────────────────────────────────────────┘
```

| If Pacc(t=0) > Pacc high and BP > BPthr | If Pacc(t=0) < Pacc low and BP < BPthr |
|---|---|
| then increase Qb and/or increase Qd and/or Qfil | then decrease Qb and/or decrease Qfil and/or start Qoa and/or increase Qd |

FIG.3

Start extracorporeal blood treatment

| | |
|---|---|
| If Pacc(t=0) > Pacc(t=1) | If Pacc(t=0) < Pacc(t=1) |
| then start Qd and/or start Qoa | then increase Qeff and Qfil |
| If Pacc(t=1) > Pacc(t=2) | If Pacc(t=1) < Pacc(t=2) |
| then increase Qd and/or increase Qoa | then maintain Qd and/or maintain Qoa |

FIG.4

Start extracorporeal blood treatment

If Cr(t=0) > Crthr

Chose a high Qd

If Cr(t=0) > Cr(t=1)
and/or
UO(t=0) > UO(t=2)

If Cr(t=0) < Cr(t=1)
and/or
UO(t=0) > UO(t=2)

then increase Qd
and/or start Qoa

then decrease Qd
and/or start Qpbp
and/or Qpre
and/or Qpost

FIG.5

**EP 4 426 368 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070055198 A
- US 20211106738 A
- CA 2824391